# EUROPEAN PATENT APPLICATION

(11) **EP 1 977 774 A2**
(43) Date of publication of application: **08.10.2008**
(21) Application number: 08154007.2
(22) Date of filing: 03.10.2005
(51) Int. Cl.: A61M 3/02

(54) **Surgical irrigator**

(30) Priority: 14.10.2004 IT PD20040252
(62) Divisional of application: 05799050.9
(71) Applicant: Bidoia s.a.s. di Gianfranco Bidoia E C., I-35010 Vigonza (Padova) (IT)
(72) Inventor: Bidoia, Gianfranco, 35142, Padova (IT)
(74) Representative: Modiano, Micaela Nadia

(57) **Abstract**

A surgical irrigator for washing body regions subjected to a procedure, provided with a containment shell (11) inside which there are means (12) for supplying a washing liquid that arrives from an associable reservoir, the containment shell (11) being constituted substantially by two distinct portions, respectively a grip portion (13) for the hand of the user and a portion (14) for ejecting the washing liquid toward the region to be washed, the irrigator being powered by a transformer (42) connected to the mains.

## Description

### Technical Field

The present invention relates to a surgical irrigator for washing body regions subjected to procedures, useful particularly but not exclusively in orthopedic surgery.

### Background Art

During surgical procedures it is often necessary to clean the operative areas in order to improve the surgeon's view and remove blood loss and other physiological residues.

Physiological residues can be portions of soft tissue, cartilage and bone fragments.

These cleaning operations sometimes need to be rather energetic in order to remove residues that have remained partially attached to the originating part.

At the same time, the energetic action of these cleaning operations can lead advantageously to limited hemostasis in the operative regions.

The need for energetic cleaning actions occurs especially in orthopedic surgery, both for prosthetic procedures and osteosynthesis procedures.

In this kinds of activity it is in fact normal to cut, drill, mill, correct, bore and file bone portions and accordingly obtain hard residues which often have substantial dimensions.

Moreover, these procedures can cause blood losses which must be eliminated or blocked at least partially if not totally.

Elimination of physiological residues and of blood losses is performed generally by means of absorbent systems or by means of washes followed by a step for aspirating the used washing liquid (in which the blood loss has been diluted and the physiological residues have been dispersed).

In order to perform hemostasis, among the various practices that are performed, it is known to spray with a certain pressure a water-based liquid (optionally with the addition of substances suitable for this purpose) on regions with limited bleeding.

It has been determined that the hemostatic effect appears to be more effective if the impulse applied to the liquid is intermittent ("pulsed"), particularly if the pause between successive impulses is substantially equal to the duration of such impulses.

The "pulsed" effect of the impulse applied to the washing liquid is more effective also in separating the physiological residues that have remained partially attached to their regions of origin.

Various devices are commercially available for performing these washing operations and in any case they all use the same functional particularities.

These devices (generally known as irrigators), which convey the sterile washing liquid into the region subjected to the procedure, can also include integrated aspiration systems.

In the case of individual irrigators, aspiration devices operating in parallel may be necessary during surgical procedures.

The characteristic shapes of known irrigation devices are substantially two: the pistol-type shape and the cylindrical (or pen-like) shape.

Both types are formed by a shell which contains pipes for conveying the washing liquid, which arrives from a reservoir, and other components.

The pistol-type shape is certainly the most ergonomic one; it has a grip portion for the hand of the user (the "butt") and a portion for ejecting the washing liquid (the "muzzle").

However, the good ergonomics of the pistol-type device contrasts with the problem of less than optimum flexibility in use, since the limited length of the ejection portion (limited by the "butt") does not allow to reach deeply into the region to be irrigated or cleaned and is poorly suited in narrow cavities.

The cylindrical shape also consists of a grip portion and an ejection portion which is arranged coaxially thereto.

This cylindrical shape is certain in less ergonomic than the pistol-type shape, due both to certain unnatural movements of the wrist during its use and to the diametrical dimensions of the grip portion, which are substantial in view of the internal components.

Small hands (such as for example female hands or hands of small-built populations) are therefore poorly suited to gripping easily large handgrips.

However, these cylindrical shapes allow to reach more easily deep regions of body cavities and to operate well in narrow cavities.

In any case, both with the pistol-type irrigation device and in the case of the cylindrical shape, the region to be irrigated/cleaned might be hidden by the device itself, making the procedure more difficult.

These irrigation devices are associated with pumping means, which generated the pulsed stream of the washing liquid.

The pumping means comprise a pump which, depending on the model of irrigator, is constituted by a peristaltic pump, to be associated externally with the irrigation device held in one hand by the surgeon, or by a reciprocating piston pump, (generally of the single-cylinder type), which is incorporated inside the shell of the irrigator itself.

The peristaltic pump is constituted by two or more eccentric rotors which press on a flexible hose, thus producing an alternated impulse of the fluid inside said hose.

Advantageously, the impulse generated by the peristaltic pump is rather energetic and can be graduated by means of controls on said pump.

This allows to size more precisely the irrigation device and particularly reduce its dimensions and therefore facilitate its use.

Further, since the peristaltic pump is external to the irrigation device, it can be reused, while said device in many cases is of the single-use type for reasons of sterility.

On the other hand, the use of the peristaltic pump entails the problem that since the hose is highly flexible in order to allow its deformation by the rotors, during the starting and stopping of the pump part of the impulse of the liquid is "lost" in the deformation along the rest of the hose, leading to a continuous and not "pulsed" stream.

Further, due to this deformation, the pressure at startup is uncontrolled and often too high.

Moreover, although these pumps are small, they encumber the area where the operators move, which is often already overloaded with equipment.

The single-cylinder reciprocating pump has a single piston in a cylindrical chamber which contains two valves, a loading valve and a discharge valve.

Advantageously, such pump can be arranged inside the device and therefore close to the ejection outlet, to which it is connected by means of rigid pipes or, if the pipes are elastic, by very short ones which therefore in practice cannot be deformed.

In this manner, the effect of continuous flow observed in the peristaltic pump when the pump starts and stops is reduced considerably.

The pulsed flow effect is certainly improved with respect to the one generated by the peristaltic pump.

The dimensions of the device, however, are larger and therefore flexibility of use in operating cavities is limited with respect to the peristaltic pump.

As regards the power supply of the pump, the peristaltic pump is associated with an electric motor which uses alternating current drawn from the centralized power supply of the operating room, therefore with a voltage which is always constant.

The pump is operated by means of a pedal which is supplied with power at low voltage.

The reciprocating pump is generally driven by an electric motor, which is obviously also integrated within the shell of the device and is supplied with power by means of batteries which are external to said device and are connected electrically by means of cables.

Batteries of course have the advantage of having a low voltage and amperage (greater safety), but on the other hand they have the problem of limited duration and of the consequent need to have a plurality of batteries available during surgical procedures (or to have a container that contains enough batteries to ensure the operation of the motor for the entire procedure).

Further, depending on their charge, the supply of power to the motor is never constant and optimum, with repercussion on the pressure of the flow of the pump.

Moreover, these batteries must be arranged in single-use containers which must be sterilized (in addition to being rather bulky).

Further, the batteries, once their life has expired, must be disposed.

Finally, it should be noted that irrigation devices which also have an aspiration system (a hose connected to an external compressor is provided in the device) all have the pedal-operated control for aspiration, thus increasing the number of pieces of equipment present in the regions where the procedure is performed.

### Disclosure of the Invention

The aim of the present invention is to provide a surgical irrigator for washing body regions subjected to a procedure, which solves the problems highlighted in known types.

Within this aim, an object of the present invention is to provide a surgical irrigator which allows a certain flexibility in movement and vision in the cavity subjected to the procedure.

Another object of the present invention is to provide a surgical irrigator which can be handled easily by the surgeon.

Another object of the present invention is to provide a surgical irrigator which allows to provide an optimum "pulsed" stream even during the starting and stopping of said device.

A further object of the present invention is to provide a surgical irrigator which is also provided with an aspiration system.

A still further object of the present invention is to provide a surgical irrigator which has a power supply which is safe for operators and is constant for the pumping means.

An object of the present invention is to provide a surgical irrigator which allows to reduce the quantity of equipment and controls provided in the region subjected to the procedure.

Another object of the present invention is to provide a surgical irrigator that can be manufactured with known systems and technologies.

This aim and these and other objects, which will become better apparent hereinafter, are achieved by a surgical irrigator for washing body regions subjected to a procedure, which has a containment shell inside which there are means for supplying a washing liquid that arrives from an associable reservoir, said shell being constituted substantially by two distinct portions, respectively a grip portion for the hand of the user and a portion for ejecting the washing liquid toward the region to be washed, characterized in that it comprises means for the articulation of said ejection portion with respect to said grip portion.

### Brief Description of the Drawings

Further characteristics and advantages of the invention will become better apparent from the following detailed description of a preferred but not exclusive embodiment thereof, illustrated by way of non-limiting example in the accompanying drawings, wherein:
Figure 1 is a side view of an irrigator according to the invention, illustrating the movement of some of its parts:
Figure 2 is a top view of the irrigator of Figure 1, illustrating the movement of some of its parts;

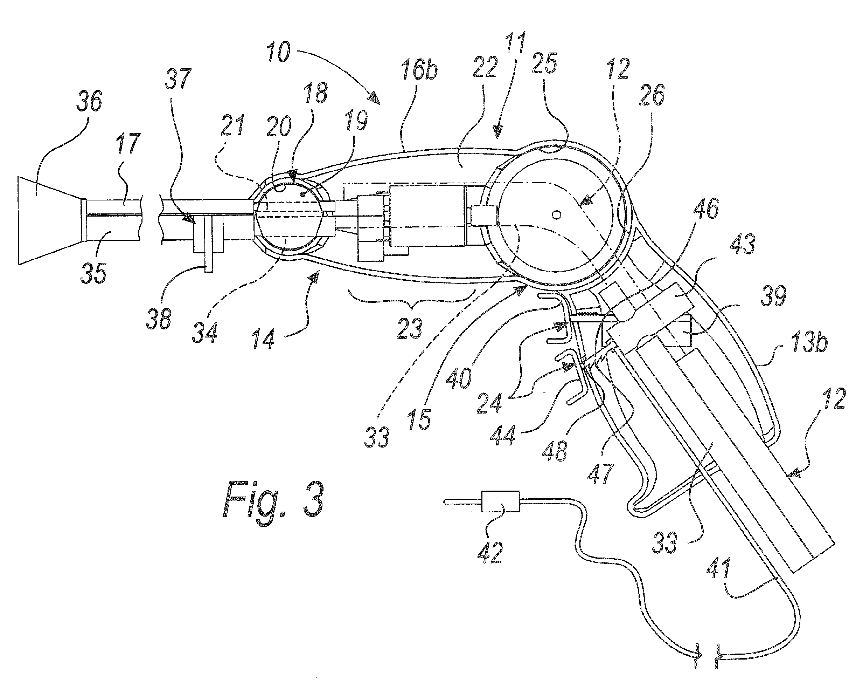

Figure 3 is a side view of the irrigator of Figure 1, with the shell open so as to illustrate the internal components;
Figure 4 is a perspective view of the irrigator of Figure 1, with the shell open so as to show the internal components.

### Ways of carrying out the Invention

With reference to the figures, a surgical irrigator for washing body regions subjected to a procedure according to the invention is generally designated by the reference numeral 10.

The surgical irrigator 10 is constituted by a containment shell 11, inside which there are means 12 for feeding the washing liquid which arrives from an associable reservoir (not shown in the figures), all of which are described in greater detail hereinafter.

The containment shell 11 is constituted substantially by two distinct portions, respectively a grip portion 13 for the hand of the user and a portion 14 for ejecting the washing liquid toward the region to be washed.

The grip portion 13 is contoured ergonomically like the butt of a pistol.

The ejection portion 14 is articulated with friction to the grip portion 13 by way of articulation means 15, described in greater detail hereinafter, about an axis which is perpendicular to the longitudinal axis of the grip portion 15.

The ejection portion 14 comprises a first body 16, which is articulated to the grip portion 13 by way of the articulation means 15, to the end of which a second body is articulated which forms a terminal ejection duct 17 for the washing liquid.

In particular, the terminal ejection duct 17 is articulated to the first body 16 by means of a spherical joint 18, which is constituted by a ball 19 arranged with friction in a corresponding complementarily shaped socket 20 at the end of the first body 16.

In other embodiments, the terminal ejection duct might not be articulated to the first body or might be articulated by means of a cylindrical hinge.

The terminal ejection duct 17 can be removable in other embodiments.

A through hole 21 is formed through the ball 19 and is coaxial and connected to the terminal ejection duct 17.

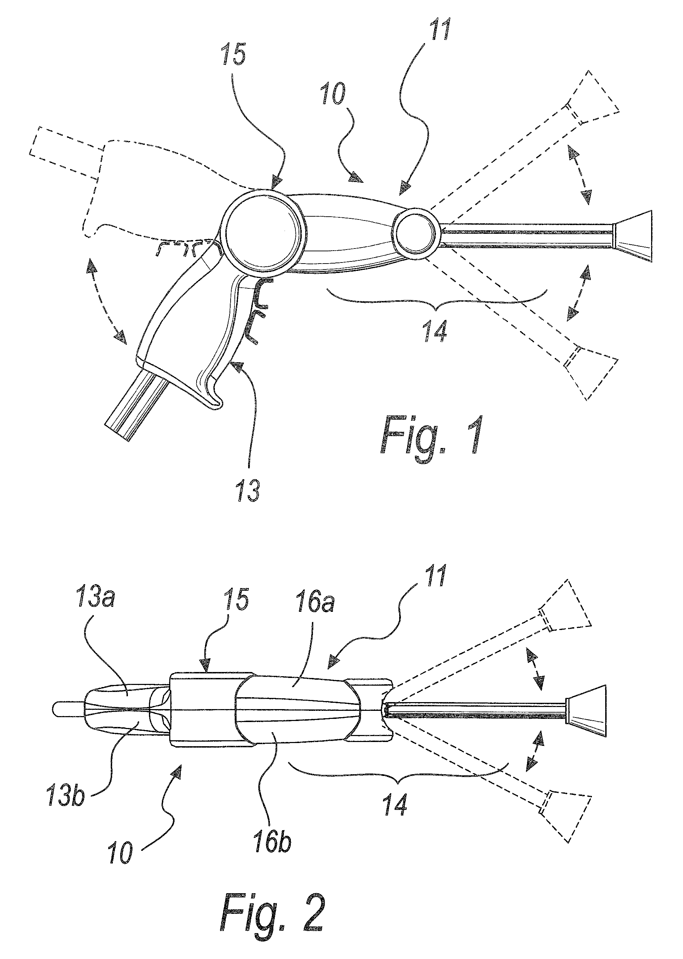

In this embodiment, the first body 16 is constituted by two first containment half-shells 16a and 16b, which are substantially mirror-symmetrical (the half-shells 16a and 16b are shown together only in Figure 2, whereas only the half-shell 16b is visible in all the other figures) and form a compartment 22 for accommodating the internal components, including pumping means 23 described hereinafter.

The grip portion 13 is constituted by two second containment half shells 13a and 13b, which are substantially mirror-symmetrical and inside which means 24 for operating the irrigator 10, also described hereinafter, are accommodated.

The articulation means 15 are constituted, in this embodiment, by a cylindrical hinge, which is formed by the cylindrical end walls 25 of the first containment half-shells 16a and 16b, on which complementarily shaped end portions 26 of the second containment half-shells 13a and 13b are engaged slidingly.

In this embodiment, the pumping means 23 are constituted by a reciprocating pump 27, for example of the single-cylinder type, which is associated with an electric motor drive 28, such as for example an electric motor which is supplied with power at low voltage.

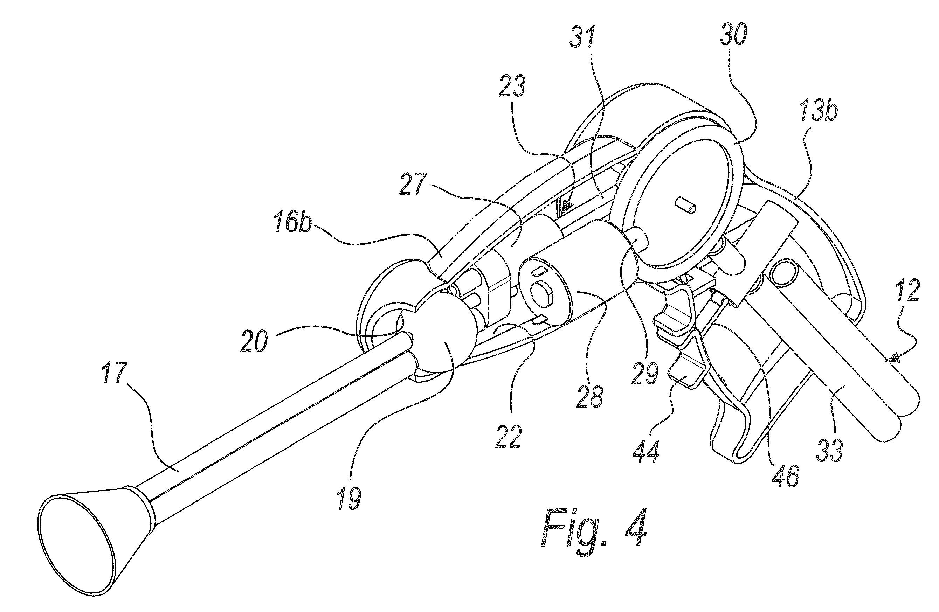

Both the reciprocating pump 27 and the electric motor drive 28 are accommodated in the compartment 22 (reference should be made in particular to Figure 4).

The kinematic connection between the electric motor drive 28 of the reciprocating pump 27 occurs for example by means of a turning pair formed by a pinion 29, which is arranged at the end of the driving shaft, and a gear 30 (the meshing teeth are not shown in the figures), which is pivoted to the second half-shells 13a and 13b.

A linkage 31 is articulated eccentrically to the gear 30 and is connected to the piston (not shown in the figures) of the reciprocating pump 27.

The means 12 for feeding the washing liquid which arrives from a reservoir, mentioned at the beginning of the description, are constituted for example by a tube 32 (shown partially in broken lines for reasons of graphical clarity), which arrives from the outside of the irrigator and is arranged through the entire grip portion 13 and the first body 16 until it enters the reciprocating pump 27 through the intake valve (not shown in the figures).

The portion of the tube 32 (not designated by a reference numeral in the figures) that passes through the cylindrical hinge that forms the articulation means 15 is flexible.

The supply means 12 further comprise a flexible hose portion (not shown in the figures), which is connected from the delivery valve (also not designated by a reference numeral in the figures) of the reciprocating pump 27 to the through hole 21 formed through the ball 19, in order to connect functionally to the terminal ejection duct 17.

Advantageously, the irrigator 10 comprises an aspiration duct 33 (partially shown schematically in broken lines), which arrives from a compressor (not shown in the figures) which is external to the irrigator; such duct is arranged through the entire grip portion 13 and the first body 16 until it is connected, by means of a through hole 34 which passes through the ball 19, to a terminal aspiration duct 35, which is parallel and adjacent to the terminal ejection duct 17.

The portion of the aspiration duct 33 (not designated by a reference numeral in the figures) that passes through the cylindrical hinge that forms the articulation means 15 is flexible, and so is the portion for connection to the hole 34 which passes through the ball 19.

A manifold 36, for example of the conical type, is provided at the end of the terminal ejection duct 17 and of the terminal aspiration duct 35.

Means 37 for filtering the aspirated material are provided on the terminal aspiration duct 35 and are suitable to block the organic residues whose dimensions are such as to block or damage the aspiration system.

These filtering means 37 are constituted for example by a guillotine device, which is provided with a gate valve 38 whose height can be adjusted along the cross-section of the terminal aspiration duct 35.

The filtering means can be of another type and can also be located in other positions along the aspiration duct 33.

As mentioned earlier, the means 24 for operating the irrigator 10 are arranged on the grip portion 13.

The operating means 24 comprise means for activating the electric motor drive 28, constituted for example by a rheostat 39 controlled by a switch 40 which can be arranged externally with respect to the second half shells 13a and 13b.

The low-voltage electric power supply is ensured by an electric cable, designated by the reference numeral 41, which reaches the rheostat 39 from a transformer 42, which can be connected to the electrical mains of the operating room, and is connected (this portion is not shown in the figures) from the rheostat to the electric motor drive 28.

Further, the control means 24 comprise means for operating the aspiration, which are arranged at the grip portion 13 and are constituted for example by a valve 43 with a flow control element which performs a translational motion and is arranged inside the second half-shells 13a and 13b and throttles the aspiration duct 33.

Such aspiration operating means also comprise a flow-rate adjustment actuator 44, which is available to the fingers of the user externally with respect to the second half-shells 13a and 13b.

The flow-rate adjustment actuator 44 comprises an element 46 for connection to the flow control element (not shown in the figures) of the valve 43; a return spring (also not shown in the figures) is provided between the bottom of the valve 43 and the flow control element.

Means for locking in intermediate adjustment positions are associated with the flow-rate adjustment actuator 44 and are constituted by a series of teeth 47 formed along the connecting element 46.

The connecting element 46 is pivoted at its end to the flow control element of the valve 43 and exits from the second half-shells 13a and 13b through a slot 48 whose dimensions are such as to make the teeth 47 protrude as well.

The operation of the invention is as follows.

First of all, the user decides the inclination of the ejection portion 14 with respect to the grip portion 13 according to the requirements.

Since the two portions are mutually articulated, it is in fact possible to decide their mutual angle in order to optimize handling, the possibility of insertion in the body cavity and the view of the washing/aspiration region.

It is also possible to adjust the inclination of the terminal ejection duct and of the terminal aspiration duct (they are rigidly coupled) with respect to the first body.

It is in fact sufficient to rotate them about any plane (they are pivoted by means of a ball).

Once the correct orientation of the irrigator has been decided, the irrigator is inserted in the body cavity.

If it is necessary to perform pulsed washing, it is sufficient to press the switch 40 of the rheostat 39 in order to activate the electric motor drive 28 and consequently the reciprocating pump 27 which propels the washing liquid in a pulsed manner into the cavity.

If aspiration is needed, it is sufficient to position the flow-rate regulation actuator 44 so as to allow the chosen aspiration flow-rate.

By pressing the flow-rate adjustment actuator 44, it is moved in steps toward the inside of the shell, consequently moving the flow control element of the valve 43 toward the bottom of said valve.

If one wishes to make the flow control element return to the initial position, it is sufficient to move outward, toward the outside of the shell, the flow-rate adjustment actuator 44, lifting it toward the switch 40 and moving the teeth 47 out of the slot 48.

In practice it has been found that the invention thus described solves the problems noted in known types of surgical irrigator; in particular, the present invention provides a surgical irrigator which allows to combine the advantages of a pistol-type shape with the advantages of a straight tubular shape.

By way of the articulation of the irrigator into at least two portions, it is in fact possible to select the preferred configuration.

In this way, there is always a grip portion that has ergonomic dimensions, since the pumping means are arranged in the ejection portion.

Further, all the controls of the irrigator are arranged in the grip portion, minimizing the accessories (control pedals) that are present in the operative region.

Further, the selected type of electric power supply has allowed to avoid the use of batteries, with evident savings in terms of space, sterilization and disposal.

Moreover, the choice to use a reciprocating pump which is arranged at the terminal ejection duct prevents the possibility of losses of pressure which would lead to a continuous stream during the starting and stopping of the pump.

Further, an irrigator has been integrated in an optimized manner with an aspirator, the controls of which are also available on the grip portion.

The invention thus conceived is susceptible of numerous modifications and variations, all of which are within the scope of the appended claims; all the details may further be replaced with other technically equivalent elements.

In practice, the materials employed, so long as they are compatible with the specific use, as well as the dimensions, may be any according to the requirements and the state of the art.

The disclosures in Italian Patent Application No. PD2004A000252 from which this application claims priority are incorporated herein by reference.

## Claims

1. A surgical irrigator for washing body regions subjected to a procedure, provided with a containment shell (11) inside which there are means (12) for supplying a washing liquid that arrives from an associable reservoir, said containment shell (11) being constituted substantially by two distinct portions, respectively a grip portion (13) for the hand of the user and a portion (14) for ejecting the washing liquid toward the region to be washed, **characterized in that** said irrigator is powered by a transformer (42) connected to the mains.

2. The irrigator of claim 1, **characterized in that** said transformer (42) is connected to operating means (24) adapted to operate the irrigator.

3. The irrigator of claim 2, **characterized in that** it comprises a motor drive (28), said operating means comprising a rheostat (39) controlled by a switch (40).

4. The irrigator of claim 3, **characterized in that** said motor drive (28) is constituted by an electric motor which is supplied at low voltage.

5. The irrigator of claim 3, **characterized in that** it comprises an electrical cable (41) which extends from said transformer (42) to said motor drive (28), said rheostat (39) being interposed on said electrical cable (41).
